# EUROPÄISCHE PATENTANMELDUNG

(11) **EP 0 542 089 A1**
(43) Veröffentlichungstag der Anmeldung: **19.05.1993**
(21) Anmeldenummer: 92118757.1
(22) Anmeldetag: 02.11.1992
(51) Int. Cl.: C12N 1/18

(54) **Verfahren und Vorrichtung zum Vermehren von Bierhefe**

(30) Priorität: 14.11.1991 DE 4137537
(71) Anmelder: KLAUS ESAU ARMATUREN MASCHINEN ANLAGEN & HANS HUEBER GmbH, D-86529 Schrobenhausen (DE)
(72) Erfinder: Sedlaczek, Klaus, W-8898 Schrobenhausen (DE)
(74) Vertreter: Schaumburg, Thoenes & Thurn

(57) **Zusammenfassung**

Bei einem Verfahren zur Vermehrung von Bierhefe, wobei eine vorgegebene Menge an steriler Bierwürze in einem Vermehrungsbehälter (10) mit Hefe versetzt und die entstehende Würze-Hefe-Suspension bei vorgegebener Temperatur mindestens zeitweilig durchmischt wird, wird die Würze-Hefe-Suspension durch einen Kreislauf (36) außerhalb des Vermehrungsbehälters (10) umgewälzt. Der Würze-Hefe-Supension wird beim Umwälzen Sterilluft und weitere Sterilwürze unter Einhaltung eines vorgegebenen Mischungsverhältnisses von Hefe-Suspension, Luft und Würze zugeführt und die erzeugte Hefe wird nach Erreichen eines Maximalvolumens im Vermehrungsgefäß (10) zur weiteren Verwendung abgeführt.

## Beschreibung

Die Erfindung betrifft ein Verfahren zum Vermehren von Bierhefe, wobei eine vorgegebene Menge an steriler Bierwürze in einem Vermehrungsbehälter mit Hefe versetzt und die entstehende Würze-Hefe-Suspension bei vorgegebener Temperatur mindestens zeitweilig durchmischt wird.

Üblicherweise wird in den Brauereien für den chargenweise ablaufenden Gärprozeß jeweils Hefe verwendet, die aus einem vorhergehenden Gärprozeß stammt. Dabei ist es bekannt, die vom vorhergehenden Gärprozeß übrig gebliebene Hefe zu reinigen und dabei beispielsweise abgestorbene Hefezellen zu entfernen, um wieder eine aktive Hefepopulation zur Verfügung zu haben.

Dieses Verfahren hat den Nachteil, daß die Hefe im Verlauf mehrerer Gärprozesse degeneriert und ihre Gärfähigkiet allmählich abnimmt, so daß sich kein optimaler Gärprozeß erhalten läßt. Nach einer gewissen Anzahl von Gärprozessen muß neue Hefe zugeführt werden.

Der Erfindung liegt die Aufgabe zugrunde, ein Verfahren der eingangs genannten Art anzugeben, durch das auf einfache Weise stets eine frische und aktive Hefepopulation in ausreichender Menge zur Verfügung gestellt werden kann.

Diese Aufgabe wird erfindungsgemäß dadurch gelöst, daß die Würze-Hefe-Suspension durch einen Kreislauf außerhalb des Vermehrungsbehälters umgewälzt wird, daß der Würze-Hefe-Suspension beim Umwälzen Sterilluft und weitere Sterilwürze unter Einhaltung eines vorgegebenen Mischungsverhältnisses von Hefe-Suspension, Luft und Würze zugeführt wird und daß die erzeugte Hefe nach Erreichen eines Maximalvolumens im Vermehrungsgefäß zur weiteren Verwendung abgeführt wird.

Dieses Verfahren bietet die Möglichkeit, Belüften und Würzedosierung nicht im Hinblick auf eine Optimierung des Gärprozesses sondern im Hinblick auf eine Optimierung der Hefevermehrung vorzunehmen. Auf diese Weise kann mit den in einer Brauerei vorhandenen Mitteln stets frische aktive Hefe in ausreichender Menge zur Verfügung gestellt werden, so daß die Vergärung jeder neuen Charge mit einer frischen Hefepopulation begonnen werden kann.

Grundsätzlich kann jeder Vermehrungszyklus durch die Zugabe von Reinzuchthefe begonnen werden. Da bei dem erfindungsgemäßen Vermehrungsverfahren aber ständig eine Erneuerung der Hefepopulation stattfindet, kann jeweils ein Restbestand eines Vermehrungszyklus für den Beginn eines neuen Vermehrungszyklus verwendet werden, ohne daß eine Degeneration der Hefezellen zu befürchten ist.

Vorzugsweise wird bei Verwendung eines Behälters mit mindestens annähernd konischem Boden die Würze-Hefe-Suspension beim Umpumpen oberhalb des Bodens entnommen und an der tiefsten Stelle des Bodens wieder in den Behälter eingeleitet, so daß der Gesamtinhalt des Behälters durch die von unten einströmende Suspension durchmischt wird. Dadurch wird verhindert, daß sich Hefezellen am Boden des Behälters absetzen, die dort mehrere Zyklen überstehen und schließlich doch altern.

Zweckmäßigerweise erfolgt das Umpumpen nicht kontinuierlich, sondern in gewissen Zeitabständen, um zwischen dem Umpumpen ein ungestörtes Hefewachstum zu ermöglichen.

Die Erfindung betrifft ferner eine Einrichtung zur Durchführung des vorstehend beschriebenen Verfahrens wobei erfindungsgemäß ein Vermehrungsbehälter zur Aufnahme einer Würze-Hefe-Suspension eine Auslaßöffnung und eine Einlaßöffnung hat, die durch eine Rohrleitung miteinander verbunden sind, in der eine Umwälzpumpe und eine mit einer Sterilluftquelle verbindbare Belüftungseinrichtung angeordnet sind, und wobei die Rohrleitung mit einem Würzebehälter verbindbar ist. Zweckmäßigerweise liegt die Verbindungsstelle der Rohrleitung mit dem Sterilwürzebehälter stromaufwärts der Umwälzpumpe, während die Belüftungseinrichtung zweckmäßigerweise stromabwärts derselben angeordnet ist.

Die Einlaßöffnung und die Auslaßöffnung sind vorteilhafterweise absperrbar, so daß zwischen den einzelnen Vermehrungszyklen zumindest der die Umwälzpumpe und die Belüftungseinrichtung umfassende Teil der Rohrleitung gereinigt werden können.

Die Dosierung der zugeführten Sterilwürze kann durch Wiegeeinrichtungen erfolgen, die mit dem Vermehrungsbehälter und/oder dem Würzetank verbunden sind. Die vorgegebene Temperatur in dem Vermehrungsbehälter und gegebenenfalls auch in dem Würzebehälter läßt sich in an sich bekannter Weise dadurch erreichen, daß diese Behälter mit geeigneten Kühl- und/oder Heißeinrichtungen versehen sind.

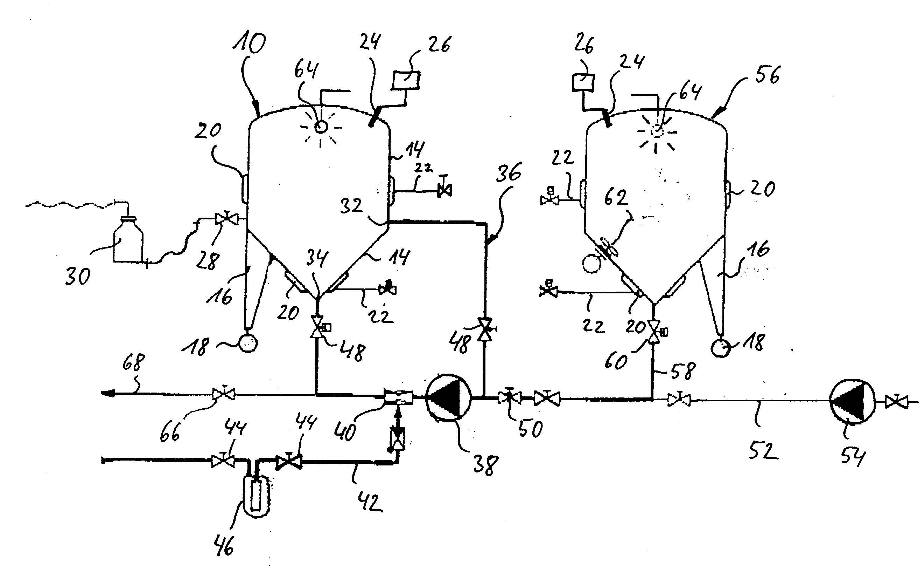

Weitere Merkmale und Vorteile der Erfindung ergeben sich aus der folgenden Beschreibung, welche in Verbindung mit der beigefügten Zeichnung die Erfindung anhand eines Ausführungsbeispieles erläutert. Die einzige Figur zeigt eine schematische Darstellung eines Hefevermehrungstankes und eines Sterilwürzevorratstankes mit den sie verbindenden Leitungen.

In der Figur ist mit 10 ein Hefevermehrungsbehälter bezeichnet, der ein konisches Unterteil 12 und ein zylindrisches Oberteil umfaßt. Der Hefevermehrungsbehälter 10 ist in an sich bekannter und daher nicht dargestellter Weise so gelagert, daß er mit einem Fuß 16 auf einer Wägezelle 18 ruht, mit der das Behältergewicht und damit der Behälterinhalt gemessen werden kann.

An der jeweiligen Außenwand von Unterteil 12 und Oberteil 14 des Hefevermehrungsbehälters 10 sind jeweils Kühltaschen 20 angeordnet, die über eine jeweilige Leitung 22 mit Kühlmittel beaufschlagt werden können, um so die Temperatur des Behälterinhaltes auf einem vorgegebenen Wert zu halten. Dabei kann die Temperatur mit einem Temperaturfühler 24 überwacht werden, der mit einem schematisch angedeuteten Überwachungsgerät 26 verbunden ist.

Der Behälter ist über ein Absperrventil 28 mit einem so genannten Karlsbergkolben 30 verbunden, über den Sterilluft und auch Reinzuchthefe zugeführt werden können.

Der Hefevermehrungstank ist über eine oberhalb des Unterteils 12 in der Wand des Oberteils 14 ausgebildete Auslaßöffnung 32 und eine an der tiefsten Stelle des konischen Unterteils liegenden Einlaßöffnung 34 an einen Rohrkreislauf 36 angeschlossen, in dem eine Umwälz- und Dosierpumpe 38 angeordnet ist, welche den Inhalt des Hefevermehrungstankes durch den Rohrkreislauf 36 umwälzen kann. Zwischen der Einlaßöffnung 34 und der Umwälzpumpe 38 ist im Rohrkreislauf 36 eine Belüftungsdüse 40 angeordnet, die über eine Leitung 42 mit einer Sterilluftquelle verbindbar ist. In der Leitung 42 ist zwischen zwei Absperrventilen 44 ein Filter 46 angeordnet. Die Teile des Rohrkreislaufes 36 zwischen der Umwälzpumpe 38 und der Auslaßöffnung 32 einerseits und der Belüftungsdüse 40 und der Einlaßöffnung 34 andererseits sind jeweils durch ein Absperrventil 48 absperrbar.

Der Rohrkreislauf 36 ist stromaufwärts der Umwälzpumpe 38 über ein Absperrventil 50 an eine Würzeleitung 52 angeschlossen, über die mittels einer Pumpe 54 Würze von einem nicht dargestellten Vorratsbehälter zugeführt werden kann. An diese Würzeleitung 52 ist ein Sterilwürze-Vorratstank 56 angeschlossen, der weitgehend gleich wie der Hefevermehrungsbehälter 10 aufgebaut ist. Gleiche Teile sind daher auch mit gleichen Bezugszeichen versehen. Der Sterilwürzevorratstank 56 weist eine einzige Zu- und Ableitung 58 auf, die an der tiefsten Stelle des konischen Unterteils 12 in den Tank mündet und durch ein Absperrventil 60 absperrbar ist.

Ferner ist an dem Sterilwürzevorratstank ein magnetisches Rührwerk 62 vorgesehen, um die Würze in dem Tank in Bewegung halten zu können.

Mit der soweit beschriebenen Vorrichtung wird das erfindungsgemäße Verfahren zur Hefevermehrung folgendermaßen durchgeführt:
Nach dem Reinigen des Hefevermehrungstankes 10 und des Sterilwürzevorratstankes 56, wobei Reinigungsmittel über eingebaute Reinigungssprühköpfe 64 in die Behälter eingeführt werden kann, werden zunächst alle Befüll-Leitungen, die mit der Würzeleitung verbunden sein sollten mit Heißwasser durchgespült. Anschließend wird das Wasser mit Würze ausgeschoben. Dann wird sterile Heißwürze in den Sterilwürzevorratstank 56 eingefüllt. Die Leitungen werden mit Heißwasser leergedrückt. Die Heißwürze wird durch Zuführen eines geeigneten Kühlmittels unter Temeperaturüberwachung und Regelung abgekühlt, wobei während der Kühlphase die Würze in dem Tank 56 mittels des Rührwerkes 62 in Bewegung gehalten wird.

In dem Hefevermehrungstank 10 wird Reinzuchthefe aus dem Labor (Karlsbergkolben) über einen Impfstutzen oder den Behältereinlauf 34 eingeführt. Anschließend wird aus dem Sterilwürzevorratsbehälter 56 die nun gekühlte Sterilwürze durch geeignete Betätigung der in den Leitungen liegenden Absperrventile durch die Einlaßöffnung 34 in den Hefevermehrungsbehälter 10 eingefüllt, bis der Würzepegel über der Auslaßöffnung 32 des Behälters 10 steht. Die Füll- oder Inhaltskontrolle erfolgt über die Wägezelle 18. Dabei wird die Temperatur der Würze in dem Behälter 10 überwacht und geregelt.

Nach Schließen des Absperrventils 50 wird das sich in dem Behälter 10 bildende Würze-Hefegemisch von Zeit zu Zeit durch den Rohrkreislauf 36 umgepumpt, um die Hefe zu belüften. Dabei wird das Gemisch an der Auslaßöffnung 32 entnommen und durch die Einlaßöffnung 34 zurückgeführt. Während des Durchströmens der Belüftungsdüse 40 wird die Hefe durch Einblasen einer vorgegebenen Menge an Sterilluft belüftet. Durch das Einströmen des Gemisches unter Druck an der Einlaßöffnung 34 wird der gesamte Inhalt des Vermehrungsbehälters 10 durchmischt und homogenisiert. Dadurch wird zuverlässig vermieden, daß sich feste Bestandteile des Gemisches am Boden absetzen und sich Bereiche bilden, in denen andere Vermehrungsbedingungen herrschen als in einem Großteil der übrigen Suspensionen. Die Luftzufuhr wird so dosiert, daß sich optimale Vermehrungsverhältnisse für die Hefezellen ergeben.

Gleichzeitig wird aus dem Sterilwürzevorratstank 56 bei laufendem Rührwerk Sterilwürze entnommen und über das Absperrventil 50 in den Rohrkreislauf 36 eingeführt, so daß entsprechend dem Zellwachstum in dem Hefevermehrungstank stets Sterilwürze zugeführt wird, um ein optimales Mischungsverhältnis aus Würze, Luft und Hefezellen sicherzustellen. Die Mengenregulierung kann über Ventile erfolgen. Die Mengenkontrolle erfolgt sowohl am Sterilwürzevorratstank 56 als auch an dem Hefevermehrungsbehälter 10 jeweils über die Wägezelle 18.

Die vorstehende Beschreibung zeigt, daß durch intensive Belüftung und stetige Sterilwürzezugabe im idealen Mischungsverhältnis von Hefe-Suspension, Luft und Sterilwürze unter Einhaltung der betriebsüblichen Temperaturen optimale Populationsraten der Hefe erreicht werden. Dabei können die Faktoren ausschließlich so bestimmt werden, daß optimale Vermehrungsverhältnisse für die Hefe erreicht werden können ohne Rücksicht auf die Qualität des gleichzeitig entstehenden Bieres. Durch die ständig wachsende dosierte Zugabe von Sterilwürze nimmt die Menge der Hefe-Suspension ebenfalls ständig zu.

Nach Erreichen des Maximalvolumens im Hefevermehrungstank 10 wird die Hefe-Suspension im Hochkräusenstadium an der Austrittsöffnung 32 aus dem Behälter 10 entnommen und mittels der Pumpe 38 nach Absperren des Ventils 48 über ein dann geöffnetes Ventil 66 und eine Abflußleitung 68 zur weiteren Verwendung abgeführt. Dabei wird die Menge wiederum über die Wegezelle 18 kontrolliert. Während des Abpumpens erfolgt nochmals eine Belüftung in der Belüftungsdüse 40, um die so erzeugte Hefe in einem optimalen Zustand für die weitere Verwendung bereitzustellen.

Im Hefevermehrungsbehälter 10 verbleibt ein Heferest, der ausreicht, um den Vermehrungsprozeß von neuem in Gang zu setzen. Nach dem Reinigen der Entleerungsleitung 68 kann der Prozeß von neuem beginnen.

## Patentansprüche

1. Verfahren zur Vermehrung von Bierhefe, wobei eine vorgegebene Menge an steriler Bierwürze in einem Vermehrungsbehälter (10) mit Hefe versetzt und die entstehende Würze-Hefe-Suspension bei vorgegebener Temperatur mindestens zeitweilig durchmischt wird, dadurch **gekennzeichnet**, daß die Würze-Hefe-Suspension durch einen Kreislauf (36) außerhalb des Vermehrungsbehälters (10) umgewälzt wird, daß der Würze-Hefe-Suspension beim Umwälzen Sterilluft und weitere Sterilwürze unter Einhaltung eines vorgegebenen Mischungsverhältnisses von Hefe-Suspension, Luft und Würze zugeführt wird und daß die erzeugte Hefe nach Erreichen eines Maximalvolumens im Vermehrungsgefäß (10) zur weiteren Verwendung abgeführt wird.

2. Verfahren nach Anspruch 1, dadurch **gekennzeichnet**, daß als Ausgangsprodukt für jeden Vermehrungszyklus Reinzuchthefe verwendet wird.

3. Verfahren nach Anspruch 1, dadurch **gekennzeichnet**, daß als Ausgangsprodukt für jeden Vermehrungszyklus (mit Ausnahme des ersten Vermehrungszyklus) eine Resthefemenge eines vorhergehenden Vermehrungszyklus verwendet wird.

4. Verfahren nach einem der Ansprüche 1 bis 4, dadurch **gekennzeichnet**, daß beim Verwenden eines Behälters (10) mit mindestens annähernd konischem Boden (12) die Würze-Hefe-Suspension beim Umpumpen oberhalb des Bodens und an der tiefsten Stelle des Bodens wieder in den Behälter (10) eingeleitet wird.

5. Verfahren nach einem der Ansprüche 1 bis 4, dadurch **gekennzeichnet**, daß der Behälterinhalt intermittierend umgepumpt wird.

6. Einrichtung zur Durchführung eines Verfahrens nach einem der Ansprüche 1 bis 5 mit einem Vermehrungsbehälter (10) zur Aufnahme einer Würze-Hefe-Suspension, dadurch **gekennzeichnet,** daß eine Auslaßöffnung (32) und eine Einlaßöffnung (34) des Vermehrungsbehälters (10) durch eine Rohrleitung (36) miteinander verbunden sind, in der eine Umwälzpumpe (38) und eine mit einer Sterilluftquelle verbindbare Lüftungseinrichtung (40) angeordnet sind und die mit einem Würzebehälter (56) verbindbar ist.

7. Einrichtung nach Anspruch 6, dadurch **gekennzeichnet**, daß der Vermehrungsbehälter (10) einen mindestens annähernd konischen Boden (12) hat und daß die Einlaßöffnung (34) an der tiefsten Stelle des Behälterbodens (12) und die Auslaßöffnung (32) oberhalb desselben angeordnet ist.

8. Einrichtung nach Anspruch 6 oder 7, dadurch **gekennzeichnet,** daß die Verbindungsstelle der Rohrleitung (36) mit dem Sterilwürzebehälter (56) stromaufwärts der Umwälzpumpe (38) liegt.

9. Einrichtung nach einem der Ansprüche 6 bis 8, dadurch **gekennzeichnet**, daß die Belüftungseinrichtung (40) stromabwärts der Umwälzpumpe (38) angeordnet ist.

10. Einrichtung nach einem der Ansprüche 6 bis 9, dadurch **gekennzeichnet,** daß die Einlaßöffnung (34) und die Auslaßöffnung (32) durch Ventile (48) absperrbar sind.

11. Einrichtung nach einem der Ansprüche 6 bis 10, dadurch **gekennzeichnet**, daß der Vermehrungsbehälter (10) und der Würzebehälter (56) mit einer Wiegeeinrichtung (18) zur Messung des Behälterinhaltes versehen sind.

12. Einrichtung nach einem der Ansprüche 6 bis 11, dadurch **gekennzeichnet**, daß der Vermehrungsbehälter (10) und der Würzebehälter (56) jeweils mit einer Kühlvorrichtung (20, 22) verbunden sind.
